# EUROPÄISCHE PATENTSCHRIFT

(11) **EP 4 262 938 B1**
(45) Veröffentlichungstag und Bekanntmachung des Hinweises auf die Patenterteilung: **01.07.2026**
(21) Anmeldenummer: 21835204.5
(22) Anmeldetag: 07.12.2021
(51) Int. Cl.: A61M 5/32, A61M 5/20, A61M 5/28, A61M 5/31

(54) **INJEKTIONSVORRICHTUNG MIT EINER KAPPE ZUR ENTFERNUNG EINER NADELSCHUTZKAPPE VON EINEM PRODUKTBEHÄLTER UND VERFAHREN ZUM VORBEREITEN EINER SOLCHEN VORRICHTUNG**
INJECTION DEVICE WITH A CAP FOR REMOVING A NEEDLE PROTECTION CAP FROM A PRODUCT CONTAINER AND METHOD FOR PREPARING SUCH A DEVICE
DISPOSITIF D'INJECTION AVEC UN CAPUCHON POUR ENLEVER UN CAPUCHON DE PROTECTION D'AIGUILLE D'UN RÉCIPIENT DE PRODUIT ET PROCÉDÉ DE PRÉPARATION D'UN TEL DISPOSITIF

(30) Priorität: 17.12.2020 CH 16062020
(43) Veröffentlichungstag der Anmeldung: 25.10.2023
(73) Patentinhaber: Ypsomed AG, 3401 Burgdorf (CH)
(72) Erfinder: ALLENSPACH, Marcel, 3400 Burgdorf (CH); HIRSCHEL, Jürg, 3007 Bern (CH); URBANEK, Leos, 3012 Bern (CH)
(74) Vertreter: Eugster, Monika Katharina
(86) Internationale Anmeldenummer: PCT/EP2021/084511
(87) Internationale Veröffentlichungsnummer: WO 2022/128606

(56) Entgegenhaltungen:
- CH-A2- 714 527
- US-A1- 2018 339 112

## Beschreibung

Die Erfindung betrifft eine Injektionsvorrichtung zur Verabreichung eines flüssigen Produkts, insbesondere eines Medikaments.

Der Begriff "Medikament" umfasst hier jede fliessfähige medizinische Formulierung, welche geeignet ist zur kontrollierten Verabreichung durch ein Mittel wie eine Kanüle oder Injektionsnadel hindurch, beispielsweise umfassend eine Flüssigkeit, eine Lösung, ein Gel, oder eine feine Suspension, welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzymen und weitere Substanzen sowohl in fester (suspendierter) oder flüssiger Form aber auch Polysaccaride, Vaccine, DNS oder RNS oder Oglionukleotide, Antikörper oder Teile von Antikörpern sowie geeignete Basis-, Hilfs- und Trägerstoffe.

Aus dem Stand der Technik sind Injektionsvorrichtungen bekannt, in denen eine Fertigspritze angeordnet ist. Die Fertigspritze weist eine Injektionsnadel auf, die unlösbar mit der Fertigspritze verbunden ist und über die ein in der Fertigspritze enthaltenes Medikament ausgegeben werden kann. Um die Injektionsnadel und das Medikament der Fertigspritze steril zu halten, wird die Injektionsnadel von einer an der Fertigspritze befestigten Nadelschutzkappe umschlossen und in Bezug auf die Umgebung steril abgedichtet. Solche Nadelschutzkappen können z.B. als sogenannte soft needle shield (SNS) oder als rigid needle shield (RNS) ausgestaltet sein. Ein rigid needle shield (RNS) weist mehrere Teile auf, insbesondere ein elastomeres kappenförmiges Teil und ein aus einem festen, d.h. nicht-elastomeren Kunststoff hergestellten hülsenförmiges Teil, welches das elastomere Teil aufnimmt und damit im Wesentlichen unlösbar verbunden ist.

Um ein in der Fertigspritze enthaltenes Medikament injizieren zu können, muss die Nadelschutzkappe von der Fertigspritze entfernt werden. Aus der CH 714 527 A2, der US 2018/339112 A1, der WO2010/136076 A1, der US 9,339,610 B2, der WO 2015/144871 A' und der US 2016/0243315 A1 ist bekannt, dass beim Abziehen eines kappenförmigen Abziehelements, das auch als Kappe bezeichnet wird, am distalen Ende der Injektionsvorrichtung angebracht ist und das distale Ende der Injektionsvorrichtung verschliesst, die an der Fertigspritze angebrachte Nadelschutzkappe mitabgezogen, d.h. beim Entfernen der Kappe von der Fertigspritze entfernt wird. Die Nadelschutzkappe verbleibt dabei in der Kappe. Hierzu weist die Kappe Eingriffsglieder auf, die beim Abziehen der Kappe in Eingriff mit der Nadelschutzkappe gebracht werden. Beim Fortsetzen der Abziehbewegung des Abziehelements nehmen die Eingriffsglieder die Nadelschutzkappe mit, wodurch diese von der Fertigspritze abgezogen wird. Um ein sicheres Abziehen der Nadelschutzkappe durch das Entfernen der Kappe zu gewährleisten, ist es aus dem Stand der Technik bekannt, dass die mit der Kappe verbundenen Eingriffsglieder in Eingriff mit der Nadelschutzkappe gelangen. Ferner ist aus der EP 2255842 B1 bekannt, dass die Nadelschutzkappe durch Drehung der Kappe von der Fertigspritze abgezogen werden kann.

Es ist eine Aufgabe der Erfindung, eine Injektionsvorrichtung und ein Verfahren zum Vorbereiten einer solchen Injektionsvorrichtung für die Verabreichung eines Produkts anzugeben, die ein einfacheres Entfernen der Nadelschutzkappe von dem Produktbehälter erlaubt.

Die Aufgabe wird mit der Injektionsvorrichtung nach Anspruch 1 gelöst Vorteilhafte Weiterbildungen ergeben sich aus den abhängigen Ansprüchen, der Beschreibung und den Figuren.

Die Erfindung geht von einer Vorrichtung zur Verabreichung eines Produkts, nämlich von einer Injektionsvorrichtung mit einer Längsachse (L), aus. Die Injektionsvorrichtung kann als so genannter Autoinjektor ausgestaltet sein, der einen Mechanismus aufweist, der ein automatisches Ausschütten des Produkts, wie z. B. durch ein Antriebselement, insbesondere eine Feder, und optional ein automatisches Einstechen und/oder Zurückziehen der Injektionsnadel bewirkt. Bei einem Autoinjektor wird die Kraft zum Ausschütten des Produkts durch das Antriebselement, wie z.B. die Feder bereitgestellt. Die Injektionsvorrichtung kann alternativ als manuelle Injektionsvorrichtung ausgestaltet sein, d.h., dass die Kraft für die Ausschüttung des Produkts durch Muskelkraft, wie z.B. durch den Benutzer selbst erfolgt. Die Injektionsvorrichtung - egal ob es sich um einen Autoinjektor oder eine manuelle Injektionsvorrichtung handelt - kann eine Nadelschutzhülse aufweisen, die nach erfolgter Injektion distal über das distale Ende der Injektionsnadel steht oder relativ zu einem Gehäuse der Injektionsvorrichtung in diese Position verschoben wird, um den versehentlichen Zugriff auf die Injektionsnadel zu verhindern und dadurch ein Verletzungsrisiko zu verringern. Optional kann die Nadelschutzhülse auch schon vor der Injektion distal über das distale Ende der Injektionsnadel hinaus stehen. Bei einem Autoinjektor kann die Nadelschutzhülse beispielsweise auch als Auslöseelement zum Auslösen der Produktausschüttung dienen, wobei die hierzu relativ zu dem Gehäuse der Injektionsvorrichtung in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors als Sichtschutz dient.

Das Antriebselement der Injektionsvorrichtung, insbesondere die Feder der Injektionsvorrichtung kann vorteilhaft die vollständige für eine automatische Produktabgabe erforderliche Energie speichern. Die Feder kann in einem energiespeichernden Zustand, also z. B. komprimiert, auseinandergezogen oder auch verdreht, in die Injektionsvorrichtung eingebaut werden und durch einen Energieabgabevorgang, also z. B. durch Entspannen, wenn die Feder komprimiert oder verdreht bzw. auf Torsion beansprucht eingebaut wurde, oder auch durch Zusammenziehen, wenn die Feder auseinandergezogen eingebaut wurde, Energie abgeben. Die Energieabgabe erfolgt vorteilhaft unmittelbar oder mittelbar, d. h. über zwischengeschaltete Bauelemente, an eine Kolbenstange oder ein Druckelement, welches auf einen Stopfen eines Produktbehälters drückt und diesen Stopfen in den Produktbehälter einschieben kann.

Die Injektionsvorrichtung weist einen Produktbehälter mit einer Injektionsnadel auf, wie z. B. eine aus dem Stand der Technik bekannte Fertigspritze oder allgemein Spritze. Der Produktbehälter kann einen z.B. hohlzylindrischen Produktbehälterabschnitt aufweisen, der einen Kolben verschiebbar lagert. Der Kolben kann mit dem Innenumfang des Produktbehälterabschnitts eine Dichtung bilden und so eine sterile Barriere bilden. Der Kolben kann z. B. mittels einer Kolbenstange der Injektionsvorrichtung in die distale Richtung verschoben werden, um über die Injektionsnadel Produkt aus dem Produktbehälter abzugeben. Die Injektionsnadel kann vorzugsweise unlösbar an dem Produktbehälter gebildet sein. Zum Beispiel kann der Produktbehälter einen Halteabschnitt, insbesondere einen Nadelhalteabschnitt, aufweisen, der distal des Produktbehälterabschnitts angeordnet ist und mit der Injektionsnadel unlösbar verbunden ist, und so zum Beispiel einen proximalen Teil der Injektionsnadel umgibt. Die Injektionsnadel kann somit von dem Halteabschnitt in die distale Richtung abragen. Der Halteabschnitt kann beispielsweise einen geringeren Aussendurchmesser als der Produktbehälterabschnitt aufweisen. Der Produktbehälterabschnitt kann sich an seinem distalen Ende zu dem Halteabschnitt hin verjüngen.

Der hierin verwendete Begriff "distal" bezieht sich auf die Richtung, in die die Spitze der Injektionsnadel zeigt. Der hierin verwendete Begriff "proximal" bezieht sich auf die Richtung, die der distalen Richtung entgegengesetzt ist.

An dem Produktbehälter, beispielsweise an dem Halteabschnitt, ist eine Nadelschutzkappe, wie z. B. ein aus dem Stand der Technik bekanntes soft needle shield (SNS) oder rigid needle shield (RNS), befestigt, insbesondere lösbar befestigt. Die Nadelschutzkappe kann z. B. reib- oder formschlüssig oder kombiniert reib- und formschlüssig auf dem Halteabschnitt befestigt sein. Die Nadelschutzkappe umschliesst die Injektionsnadel und dichtet sie in Bezug auf die Umgebung steril ab. Ein soft needle shield (SNS) umfasst ein oder besteht aus einem Elastomer, beispielsweise auf Gummi- oder Kautschukbasis gebildeten Teil, welches die Nadel umgibt. Das soft needle shield (SNS) weist an seinem Aussenumfang eine weiche, wie z. B. aus einem gummi- oder kautschukartigen Material gebildete Oberfläche auf. Ein rigid needle shield (RNS) weist zumeist mehrere Teile auf, insbesondere ein elastomeres kappenförmiges inneres Teil und ein aus einem steiferen, d. h. typischerweise nicht-elastomeren Kunststoff hergestelltes hülsenförmiges oder kappenförmiges äusseres Teil, welches das elastomere Teil aufnimmt und damit im Wesentlichen unlösbar verbunden ist. Das äussere hülsen- oder kappenförmige Teil umgibt das innere kappenförmige Teil und ist mit der inneren Kappe beispielsweise unlösbar verbunden, so dass die äussere und innere Kappe eine Einheit bilden. Das äussere Teil kann aus einem härteren Kunststoff als das innere Teil gebildet sein. Das äussere Teil kann beispielsweise aus Polyethylen, Polystyrol, Polypropylen oder einem anderen geeigneten Kunststoff sein. Das innere Teil kann beispielsweise aus Gummi oder Kautschuk oder einem anderen geeigneten Material gebildet sein.

An dem distalen Ende der Injektionsvorrichtung oder des Gehäuses, wie z. B. eines Aufnahmegehäuses der Injektionsvorrichtung kann eine Kappe, die auch als Verschlusskappe oder Abziehkappe bezeichnet werden oder ausgestaltet sein kann, befestigt sein und das distale Ende des Gehäuses oder des Aufnahmegehäuses verschliessen. Die Kappe ist mehrteilig ausgebildet. Die Injektionsvorrichtung umfasst ein Gehäuse, wie z.B. ein Aufnahmegehäuse der Injektionsvorrichtung zur Aufnahme des Produktbehälters, wobei der Produktbehälter eine fest verbundene Injektionsnadel aufweist und wobei an dem Produktbehälter die Nadelschutzkappe lösbar angeordnet ist. Die Nadelschutzkappe umschliesst die Injektionsnadel und dichtet die Injektionsnadel gegenüber der Umgebung steril ab. Die Kappe kann z. B. mit dem Gehäuse oder Aufnahmegehäuse und/oder mit der Nadelschutzhülse reib- und/oder formschlüssig verbunden sein, wie z. B. verschnappt sein. Die Kappe kann vorzugsweise aus Kunststoff gebildet sein. Alternativ kann die Kappe aus Metall gebildet sein. Die Kappe kann z. B. während des Entfernens von der Injektionsvorrichtung und/oder dem Gehäuse und/oder der Nadelschutzhülse mit einer Axialbewegung oder mit einer kombinierten/seriellen Axial-Dreh-Bewegung von der Injektionsvorrichtung, wie z. B. dem Gehäuse oder Aufnahmegehäuse und/oder Nadelschutzhülse, abnehmbar sein.

Die Injektionsvorrichtung kann ferner einen Produktbehälterhalter umfassen, welcher mit dem Gehäuse der Injektionsvorrichtung fest, insbesondere axial- und drehfest verbunden ist. Der Produktbehälterhalter kann zur Aufnahme des Produktbehälters dienen, wobei in dem Produktbehälterhalter der Produktbehälter fest, insbesondere axial- und vorzugsweise drehfest gehalten werden kann. Alternativ können das Gehäuse und der Produktbehälterhalter einteilig ausgebildet sein. Alternativ kann der Produktbehälterhalter relativ zu dem Gehäuse axial bewegbar und/oder drehbar angeordnet sein.

Die Kappe, welche lösbar an dem distalen Ende des Gehäuses oder am distalen Ende der Nadelschutzhülse der Injektionsvorrichtung vorgesehen ist, ist mehrteilig ausgebildet. Die Kappe umfasst eine Haltehülse, eine Drehhülse und ein oder mehrere Eingriffselemente, um beim Entfernen der Kappe von der Injektionsvorrichtung das Entfernen der Nadelschutzkappe von dem Produktbehälter zu bewirken. Die Kappe, welche mit dem Eingriffselement gekoppelt ist, kann über das Eingriffselement derart mit der Nadelschutzkappe verbindbar sein, dass das Entfernen der Kappe von der Injektionsvorrichtung das Entfernen der Nadelschutzkappe von dem Produktbehälter bewirkt. Insbesondere kann zumindest ein Teil der Bewegung oder die gesamte Bewegung der Kappe in die distale Richtung auf das Eingriffselement übertragen werden, d. h., dass das Eingriffselement von der Kappe mitgenommen wird, so dass das Eingriffselement die Nadelschutzkappe von dem Produktbehälter, insbesondere dem Halteabschnitt, abzieht.

In einer Ausführungsform kann das Eingriffselement derart verformbar sein, dass das Eingriffselement von einer beabstandeten Position, in welcher das Eingriffselement von der Nadelschutzkappe radial beabstandet ist, in eine Eingriffsposition, in welcher das Eingriffselement in Eingriff mit der Nadelschutzkappe ist, bringbar ist, wobei das Eingriffselement beim Entfernen der Kappe verformt wird. Das Eingriffselement kann z. B. im Auslieferungszustand der Injektionsvorrichtung in Bezug auf die Nadelschutzkappe in der beabstandeten Position sein. In der Eingriffsposition ist das Eingriffselement in Bezug auf die Nadelschutzkappe derart angeordnet, dass eine Bewegung der Kappe in die distale Richtung eine Mitnahme der Nadelschutzkappe bewirkt und somit die Nadelschutzkappe von dem Produktbehälter entfernt wird. In der Eingriffsposition des Eingriffselements greift das Eingriffselement an oder in die Nadelschutzkappe. Das Eingriffselement kann an oder in eine Mantelfläche oder an oder in eine Kante oder an oder in eine distalen Stirnfläche oder an oder in eine proximale Stirnfläche der Nadelschutzkappe greifen. Das Eingriffselement kann einen Haken oder mehrere Haken umfassen. Besonders bevorzugt kann das Eingriffselement zumindest teilweise hakenförmig ausgebildet sein. Besonders bevorzugt kann das Eingriffselement ferner hülsenförmig oder zylinderförmig ausgebildet sein. Das hakenförmige Eingriffselement kann einen kurzen und einen langen Schenkel aufweisen. Vorzugsweise kann der lange Schenkel verformbar ausgebildet sein. Ferner kann der kurze Schenkel zahn- oder dreieckförmig oder spitzwinklig ausgebildet sein. Alternativ kann das Eingriffselement eine andere Ausgestaltung aufweisen, wobei in der beabstandeten Position des Eingriffselements das Eingriffselement von der Nadelschutzkappe radial beabstandet ist und in der Eingriffsposition des Eingriffselements das Eingriffselement in Eingriff mit der Nadelschutzkappe ist, wobei das Eingriffselement beim oder vor dem Entfernen der Kappe verformt wird.

In der beabstandeten Position des Eingriffselements kann das Eingriffselement unverformt, verformt oder radial nach aussen verformt sein. In der Eingriffsposition des Eingriffselements kann das Eingriffselement unverformt, verformt oder radial nach innen verformt sein. Das Eingriffselement kann vorzugsweise plastisch und/oder elastisch verformbar sein.

Als eine plastische Verformung wird eine bleibende Verformung bezeichnet. Die Verformung eines Materials ist plastisch, wenn das Material nicht wieder von allein seine ursprüngliche Form annimmt. Nach Einwirkung von einer Kraft oder Belastung auf das Material behält das Material seine Form bei. Als eine elastische Verformung wird eine reversible Verformung bezeichnet. Dabei nimmt ein Material nach Einwirkung einer Kraft oder einer Belastung auf das Material wieder seine ursprüngliche Form an.

Das Eingriffselement ist vorzugsweise aus Metall, insbesondere aus Stahl, besonders bevorzugt aus rostfreiem Stahl oder Federstahl gebildet. Das Eingriffselement ist aus einem Material gebildet, welches eine Biegefestigkeit aufweist, welche eine plastische und/oder elastische Verformung zulässt. Besonders bevorzugt ist das Eingriffselement derart ausgebildet, dass es in der beabstandeten Position des Eingriffselements plastisch und/oder elastisch verformt und in der Eingriffsposition plastisch und/oder elastisch unverformt ausgebildet ist oder, dass es in der beabstandeten Position des Eingriffselements plastisch und/oder elastisch unverformt und in der Eingriffsposition plastisch und/oder elastisch verformt ausgebildet ist.

Ferner kann an dem Gehäuse oder an einem Gehäuse fest verbundenen Teil ein oder mehrere Sperrelemente vorgesehen sein, wobei in der Eingriffsposition das oder die Sperrelemente das Eingriffselement in Eingriff mit der Nadelschutzkappe hält oder bringt. Das Sperrelement kann eine erste und/oder eine zweite schräge Fläche, insbesondere eine erste und/oder eine zweite nach innen ragende schräge Fläche, umfassen. Die erste und/oder die zweite schräge Fläche des Sperrelements können eine Neigung aufweisen. Die erste und die zweite schräge Fläche können zueinander geneigt sein. Das Eingriffselement der Kappe kann derart mit dem Sperrelement des Gehäuses gekoppelt sein, dass während dem Entfernen der Kappe von der Injektionsvorrichtung das Eingriffselement relativ zu der Nadelschutzkappe bewegbar ist oder bewegt wird und bei dieser Bewegung, insbesondere einer Axialbewegung, mittels dem Sperrelement des Gehäuses derart verformbar ist oder verformt wird, dass das Eingriffselement, insbesondere der kurze Schenkel des Eingriffselements in Eingriff mit der Nadelschutzkappe gelangbar ist oder gelangt. In der Eingriffsposition des Eingriffselements ist das Eingriffselement mit der Nadelschutzkappe axialfest verbunden, wobei die Nadelschutzkappe von dem Eingriffselement der Kappe bei der Fortführung der Axialbewegung der Kappe mitgenommen wird. Mit anderen Worten umfasst der Hub, den die Kappe beim Entfernen von der Injektionsvorrichtung relativ dem Gehäuse entlang der Längsachse (L) in die distale Richtung ausführt, einen ersten Teilhub, während dem die Kappe relativ zu der Nadelschutzkappe bewegbar ist oder bewegt wird, und einen zweiten Teilhub, während dem die Nadelschutzkappe die Bewegung der Kappe mitmacht oder von der Kappe mitgenommen wird.

In einer alternativen Ausführungsform kann ein oder mehrere Eingriffselemente vorgesehen sein, welche elastisch und/oder plastisch verformbar ausgebildet sind, wobei das oder die mehreren Eingriffselemente immer und/oder bereits beim Montieren einer Injektionsvorrichtung für die Verabreichung eines Produkts in einem Kontakt mit der Nadelschutzkappe sind. Dieses Montageverfahren umfasst ferner das Verschieben oder Einsetzen eines Produktbehälters mit der lösbar verbundenen Nadelschutzkappe in ein Gehäuse entlang einer Längsachse (L) in die distale Richtung, wobei das Gehäuse an einem distalen Ende eine Kappe aufweist. Eine Mantelaussenfläche der Nadelschutzkappe gleitet dabei axial über das oder die Eingriffselemente, insbesondere den oder die kurzen Schenkel des Eingriffselements. In der Position, in welcher der Produktbehälter in dem Gehäuse eingesetzt ist, sind das oder die Eingriffselemente der Kappe in einer Eingriffsposition. In dem Auslieferungszustand der Injektionsvorrichtung können somit das oder die Eingriffselemente der Kappe in einer Eingriffsposition sein.

In einer alternativen Ausführungsform kann die Kappe mit einem oder mit mehreren Eingriffselementen relativ zu einer Nadelschutzkappe in die proximale Richtung bewegt werden, um die Kappe auf ein Produktbehälter aufgenommenes Gehäuse aufzusetzen.

Die Mantelfläche der Nadelschutzkappe kann eine oder mehrere Öffnungen oder ein oder mehrere Befestigungsmittel aufweisen, in welche das Eingriffselement in der Eingriffsposition des Eingriffselements eingreifen oder sich einbohren kann. Alternativ weist die Nadelschutzkappe keine Öffnung oder kein Befestigungsmittel auf, wobei das Eingriffselement in der Eingriffsposition des Eingriffselements in die Mantelfläche der Nadelschutzkappe eingreifen oder sich einbohren kann.

Die Kappe kann vorzugweise an dem distalen Ende des Gehäuses der Injektionsvorrichtung vorgesehen sein. Die Kappe ist mehrteilig ausgebildet. Die Kappe umfasst eine Haltehülse, eine Drehhülse und ein oder mehrere Eingriffselemente. Die Haltehülse dient zum Greifen der Kappe. Der Benutzer kann die Kappe an der Haltehülse greifen, insbesondere in die distale Richtung ziehen oder bewegen, um die Kappe von der Injektionsvorrichtung zu entfernen. Die Drehhülse ist teilweise von der Haltehülse aufgenommen. Die Drehhülse ist relativ zu dem Gehäuse der Injektionsvorrichtung und relativ zu der Haltehülse drehbar angeordnet. Die Haltehülse ist relativ zu dem Gehäuse axial bewegbar und drehfest angeordnet. Ferner ist das oder die mehreren Eingriffselemente axialfest zu der Drehhülse angeordnet, wobei das oder die mehreren Eingriffselemente axial zu dem Gehäuse bewegbar angeordnet ist, um beim Entfernen der Kappe von der Injektionsvorrichtung das Entfernen der Nadelschutzkappe von dem Produktbehälter zu bewirken. Besonders bevorzugt ist das Eingriffselement axialfest und drehbar zu der Drehhülse angeordnet. Alternativ können das Eingriffselement und die Drehhülse axial- und drehfest ausgebildet sein.

Ferner umfasst die Injektionsvorrichtung ein erstes Element, welches an dem Gehäuse vorgesehen ist. Das erste Element ist in einem ersten Führungseingriff mit einem an der Drehhülse vorgesehenen ersten Gegenelement. Des Weiteren umfasst die Injektionsvorrichtung ein zweites Element, welches an der Haltehülse vorgesehen ist. Das zweite Element ist in einem zweiten Führungseingriff mit einem an der Drehhülse vorgesehenen zweiten Gegenelement, wobei der erste und der zweite Führungseingriff derart ausgebildet sind, dass beim Entfernen der Kappe von der Injektionsvorrichtung das Entfernen der Nadelschutzkappe von dem Produktbehälter bewirkt wird.

Bevorzugt sind das Element und/oder das Gegenelement als Führungskulisse oder Führungsnocke ausgebildet. Besonders bevorzugt kann die Führungskulisse ein Gewinde umfassen oder gewindeförmig ausgebildet sein. Gemäß der beanspruchten Erfindung sind der erste und der zweite Führungseingriff als Gewindeeingriffe ausgebildet.

Des Weiteren können bevorzugt das erste und das zweite Gegenelement der Drehhülse als zueinander gegenläufige Gewinde ausgebildet sein.

Ferner können bevorzugt das erste und das zweite Element als Führungsnocke ausgebildet sein.

Besonders bevorzugt sind der erste und/oder der zweite Gewindeeingriff als nicht-selbsthemmende Gewindeeingriffe ausgebildet. Gemäß der beanspruchten Erfindung weisen der erste und der zweite Gewindeeingriff unterschiedliche Steigungen auf.

Die unterschiedlichen Steigungen dienen der Einstellung der Übersetzung oder Untersetzung der Haltehülse relativ zu der Drehhülse und/oder des Eingriffselements. Somit kann vorzugsweise durch einen längeren axialen Weg der Haltehülse in die distale Richtung eine höhere Kraft auf die Drehhülse und/oder auf das Eingriffselement übertragen werden. Somit kann der Benutzer die Kappe mit einem kleineren Kraftaufwand von der Injektionsvorrichtung entfernen.

Die Kappe kann relativ zu dem Gehäuse oder/und zu der Nadelschutzhülse axial bewegbar sein. Diese relative axiale Bewegung kann zum Entfernen der Kappe von der Injektionsvorrichtung dienen. Dabei kann durch die relative axiale Bewegung die Kappe entfernt werden oder ermöglichen, dass die Kappe entfernt werden kann. Bei dem Entfernen der Kappe von der Injektionsvorrichtung werden die Haltehülse und/oder die Drehhülse in axiale Richtung, insbesondere in die distale Richtung bewegt. Durch den ersten und/oder zweiten Gewindeeingriff, insbesondere der gegenläufigen Gewindeeingriffe und der unterschiedlichen Steigungen findet eine Weg- und Kraftübersetzung-/untersetzung statt. Besonders bevorzugt kann die Steigung des zweiten Gewindeeingriffs grösser als die Steigung des ersten Gewindeeingriffs sein. Besonders bevorzugt kann die Steigung des ersten Gewindeeingriffs kleiner als die Steigung des zweiten Gewindeeingriffs sein. Der axiale Weg der Haltehülse kann dabei vorzugsweise auf einen kürzeren axialen Weg der Drehhülse übersetzt werden, wobei die Kraft der Drehhülse somit erhöht wird.

Alternative erste und/oder zweite Führungseingriffe können vorgesehen sein. Die Haltehülse und die Drehhülse können vorzugsweise über den ersten und/oder den zweiten Führungseingriff derart gekoppelt sein, dass beim Entfernen der Kappe von der Injektionsvorrichtung die Haltehülse einen längeren axialen Weg als die Drehhülse und/oder das Eingriffselement macht. Besonders bevorzugt kann das Eingriffselement axialfest und drehbar zu der Drehhülse angeordnet sein. Durch die Weg- und Kraftübertragung kann besonders bevorzugt somit durch den längeren axialen Weg der Haltehülse eine grössere Kraft auf die Drehhülse übertragen werden. Die Drehhülse kann bevorzugt durch die axialfeste Anordnung zu dem Eingriffselement diese grössere Kraft auf das Eingriffselement übertragen.

Die Kappe, insbesondere die Haltehülse und/oder die Drehhülse können vorzugsweise aus Kunststoff gebildet sein. Alternativ können die Haltehülse und/oder die Drehhülse aus Metall gebildet sein.

Ferner kann die Injektionsvorrichtung, insbesondere die Kappe, die Haltehülse, die Drehhülse und/oder das Gehäuse eine visuelle Markierung, insbesondere in Form eines Zeichens, besonders bevorzugt in Form eines Pfeils aufweisen, um anzuzeigen in welche axiale Richtung die Kappe von der Injektionsvorrichtung entfernt werden soll. Diese Markungen dient der einfacheren Bedienung der Injektionsvorrichtung.

Ergänzend wird auf die Merkmale, die im Zusammenhang mit der hierin beschriebenen Vorrichtung offenbart werden, verwiesen, die auch das Verfahren vorteilhaft weiterbildet.
- Figur 1: eine Explosionsansicht eines distalen Endes einer erfindungsgemässen Injektionsvorrichtung mit einer Längsachse (L).
- Figur 2: eine Längsschnittansicht der Injektionsvorrichtung gemäss Figur 1, wobei die Kappe an einem distalen Ende der Injektionsvorrichtung angeordnet ist.
- Figur 3: die Injektionsvorrichtung gemäss Figur 2, wobei die Injektionsvorrichtung um 90 Grad um die Längsachse (L) gedreht ist.
- Figur 4: eine Perspektivenansicht einer Drehhülse (2) der Kappe.
- Figur 5: eine Längsschnittansicht der Kappe gemäss Figur 1, wobei die Kappe von der Injektionsvorrichtung abgenommen ist und die Nadelschutzkappe (6a) hält.
- Figur 6: eine Längsschnittansicht der Injektionsvorrichtung gemäss Figur 1, wobei die Kappe von der Injektionsvorrichtung abgenommen ist und somit nicht ersichtlich ist.

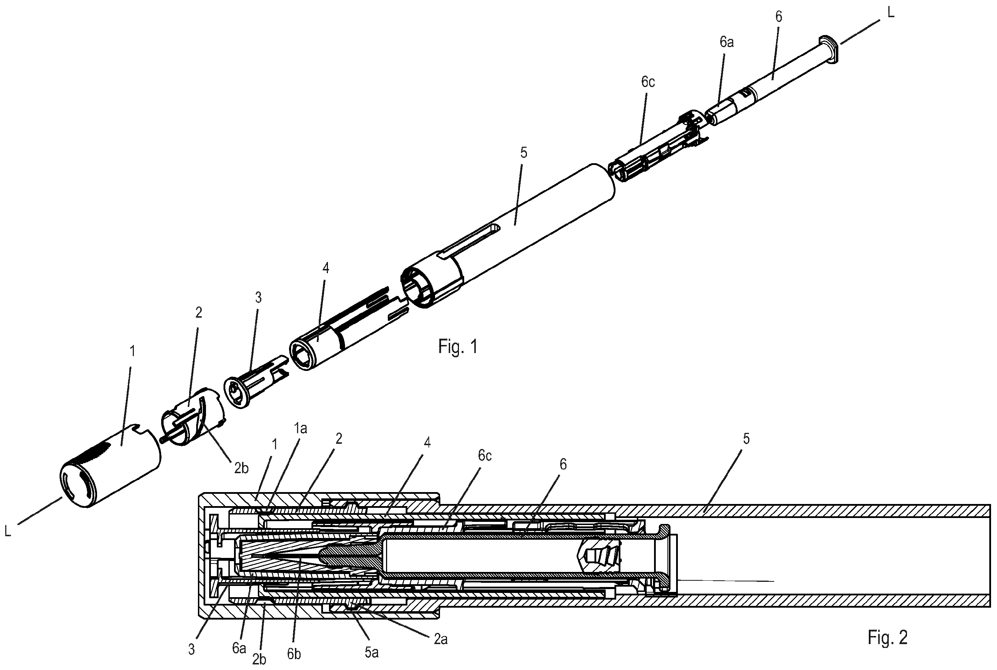

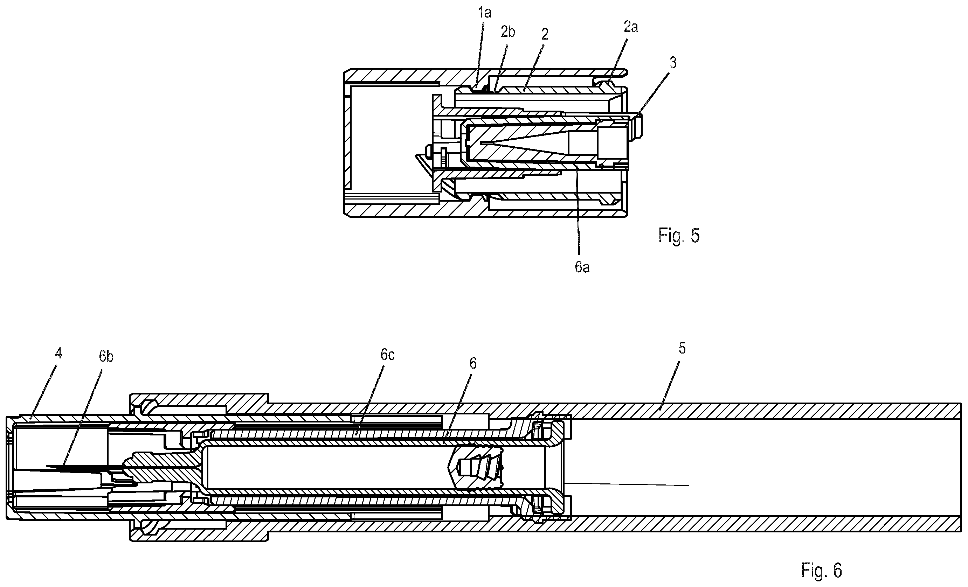

In der Figur 1 ist eine Explosionsansicht einer erfindungsgemässen Injektionsvorrichtung ersichtlich, wobei eine Kappe lösbar an der Injektionsvorrichtung angeordnet ist. Die Injektionsvorrichtung kann beispielsweise in einem Auslieferungszustand die Kappe an dem distalen Ende aufgesetzt haben. Die Kappe umfasst eine Haltehülse (1), eine Drehhülse (2) und ein oder mehrere Eingriffselemente (3). Die Injektionsvorrichtung umfasst ein Gehäuse (5). Das Gehäuse (5) kann als hülsenförmiges, insbesondere zylindrisches Aufnahmegehäuse mit einem distalen und einem proximalen Teil ausgebildet sein. Das Gehäuse (5) dient zur Aufnahme eines Produktbehälters (6), welcher über den Produktbehälterhalter (6c) im Gehäuse gehalten wird. Der Produktbehälter (6) weist eine fest verbundene Injektionsnadel (Figur 2; 6b) auf, wobei an dem Produktbehälter (6) eine Nadelschutzkappe (6a) lösbar angeordnet ist, welche die Injektionsnadel (Figur 2; 6b) umschliesst und gegenüber der Umgebung steril abdichtet. Die Injektionsvorrichtung umfasst ferner eine Nadelschutzhülse (4). Die Nadelschutzhülse (4) kann relativ zu dem Gehäuse (5) der Injektionsvorrichtung zum Auslösen einer Produktausschüttung in die proximale Richtung verschiebbar sein. Die Nadelschutzhülse (4) ist vorzugsweise drehfest mit dem Gehäuse (5) verbunden. Nach erfolgter Produktausschüttung kann die Nadelschutzhülse (4) relativ zu dem Gehäuse (5) in die distale Richtung verschiebbar sein, um die Spitze der Injektionsnadel (Figur 2; 6b) abzudecken, um eine Verletzungsgefahr zu verringern. Die Kappe umfasst ein oder mehrere Eingriffselemente (3). Das Eingriffselement (3) dient beim Entfernen der Kappe von der Injektionsvorrichtung dazu, das Entfernen der Nadelschutzkappe (6a) von dem Produktbehälter (6) zu bewirken. Dazu ist das Eingriffselement (3) hakenförmig ausgebildet oder weist einen oder mehrere Haken auf. In dieser Ausführungsform ist das Eingriffselement (3) hülsenförmig ausgebildet und weist einen oder mehrere Haken auf. Andere Ausgestaltungen können vorgesehen sein. Der Haken ist derart ausgebildet, dass der Haken in oder an die Nadelschutzkappe (6a) greifen kann, um in einer Eingriffsposition mit der Nadelschutzkappe (6a) zu sein oder zu gelangen. Das Eingriffselement (3) ist vorzugsweise aus Metall, insbesondere aus Stahl, insbesondere bevorzugt aus rostfreiem Stahl oder rostfreiem Federstahl gebildet. Alternativ kann das Eingriffselement (3) aus Kunststoff gebildet sein. Das Eingriffselement (3) ist vorzugsweise axialfest und drehbar zu der Drehhülse (2) angeordnet. Dazu kann die Drehhülse (2) ein oder mehrere Eingriffselemente (3) umfassen, welche das Eingriffselement (3) axialfest, insbesondere axialfest und drehbar zu der Drehhülse (2) verbinden. Die Haltehülse (1) und/oder die Drehhülse (2) sind vorzugsweise aus Kunststoff gebildet. Alternativ können die Drehhülse (2) und das Eingriffselement (3) einstückig ausgebildet sein und vorzugsweise aus Metall, insbesondere aus Stahl, besonders bevorzugt aus rostfreiem Stahl oder rostfreiem Federstahl gebildet sein. Die Kappe ist vorzugsweise direkt an dem distalen Ende des Gehäuses (5) lösbar vorgesehen. Alternativ oder zusätzlich kann die Kappe über die Nadelschutzhülse (3) an dem distalen Ende des Gehäuses (4) lösbar vorgesehen sein. Ferner umfasst das Gehäuse (5) ein erstes Element (Figur2; 5a), welches in einem ersten Führungseingriff mit einem an der Drehhülse (2) vorgesehenen ersten Gegenelement (Figur 2; 2a) ist. Des Weiteren ist ein zweites Element (Figur 2; 1a) an der Haltehülse (1) vorgesehen, welches in einem zweiten Führungseingriff mit einem an der Drehhülse (2) vorgesehenen zweiten Gegenelement (2b) ist, wobei der erste und der zweite Führungseingriff derart ausgebildet sind, dass beim Entfernen der Kappe von der Injektionsvorrichtung das Entfernen der Nadelschutzkappe (6a) von dem Produktbehälter (6) bewirkt wird. Besonders bevorzugt sind der erste und der zweite Führungseingriff als Gewindeeingriffe, insbesondere als gegenläufige Gewindeeingriffe, besonders bevorzugt als gegenläufige Gewindeeingriffe mit unterschiedlicher Steigung ausgebildet. Die Haltehülse (1) ist derart ausgebildet, dass sie durch den Benutzer greifbar ist. Dazu kann die Haltehülse (1), insbesondere an einer Mantelaussenfläche der Haltehülse (1) ein oder mehrere Greifelemente umfassen, welche dazu dienen, dass der Benutzer die Kappe von der Injektionsvorrichtung entfernen kann, wobei die Haltehülse relativ zu dem Gehäuse axial bewegbar und drehfest angeordnet ist. Um die Kappe von der Injektionsvorrichtung zu entfernen kann der Benutzer die Haltehülse (1) in die distale Richtung bewegen oder ziehen. Die Drehhülse (2) ist zumindest teilweise von der Haltehülse (1) aufgenommen. Die Drehhülse (2) ist relativ zu dem Gehäuse (5) und relativ zu der Haltehülse (1) drehbar angeordnet.

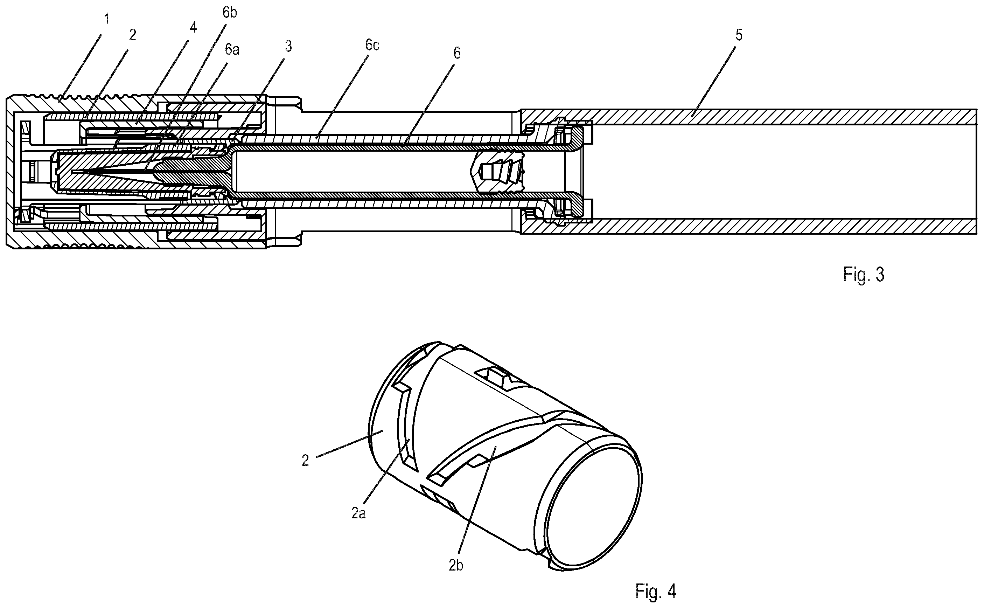

In der Figur 2 ist eine Längsschnittansicht der Injektionsvorrichtung gemäss Figur 1 dargestellt, wobei die Kappe an einem distalen Ende der Injektionsvorrichtung angeordnet ist. Das Eingriffselement (3) der Kappe ist in der Eingriffsposition, wie in der Figur 3 dargestellt ist. Das Eingriffselement (3), insbesondere ein kurzer Schenkel des Eingriffselements (3) greift an die Kante der Nadelschutzkappe (6a). In diesem Ausführungsbeispiel sind das erste Element (5a) des Gehäuses (5) als Führungsnocke und das erste Gegenelement (2a) der Drehhülse (2) als Führungskulisse und das zweite Element (1a) der Haltehülse (1) als Führungsnocke und das zweite Gegenelement (2b) der Drehhülse (2) als Führungskulisse ausgebildet. Alternative Ausgestaltungen des ersten und/oder zweiten Führungseingriffs können vorgesehen sein. Besonders bevorzugt sind der erste (5a; 2a) und der zweite Führungseingriff (1a; 2b) als Gewindeeingriffe, insbesondere als gegenläufige Gewindeeingriffe, besonders bevorzugt als gegenläufige Gewindeeingriffe mit unterschiedlicher Steigung ausgebildet. Besonders bevorzugt kann die Steigung des zweiten Gewindeeingriffs (1a; 2b) grösser als die Steigung des ersten Gewindeeingriffs (5a; 2a) sein, um eine geeignete Weg- und Kraftübersetzung-/untersetzung zu erzielen.

Um die Kappe von der Injektionsvorrichtung zu entfernen, zieht oder bewegt der Benutzer die Kappe, insbesondere die Haltehülse (1) axial entlang der Längsachse (L) relativ zu dem Gehäuse (5) und/oder zu der Nadelschutzhülse (4) in die distale Richtung. Dazu kann eine visuelle Markierung, insbesondere in Form eines Zeichens an der Injektionsvorrichtung vorgesehen sein. Dabei dreht die Drehhülse (2) relativ zu der Haltehülse (1) und dem Gehäuse (5). Die Haltehülse (1) und die Drehhülse (2) sind über den ersten und/oder den zweiten Gewindeeingriff derart gekoppelt sind, dass beim Entfernen der Kappe von der Injektionsvorrichtung die Haltehülse (1) einen längeren axialen Weg, insbesondere in die distale Richtung als die Drehhülse (2) macht.

Durch eine Axialbewegung, insbesondere in die distale Richtung der Kappe zu dem Gehäuse (5) und/oder zu der Nadelschutzhülse (4) kann die Kappe von der Injektionsvorrichtung entfernt werden. Dabei kann das Eingriffselement (3) der Kappe, insbesondere über die axialfeste Verbindung zwischen dem Eingriffselement (3) und der Drehhülse (2) in die distale Richtung bewegt werden. Das Eingriffselement (3) der Kappe kann dabei gleichzeitig in die distale Richtung bewegt werden. Das Eingriffselement (3) ist oder wird in Eingriff mit der Nadelschutzkappe (6a) gebracht. Besonders bevorzugt greift das Eingriffselement (3), insbesondere der kurze Schenkel des Eingriffselements (3) an die Kante der Nadelschutzkappe (6a).

Das Entfernen der Kappe von der Injektionsvorrichtung bewirkt das Entfernen der Nadelschutzkappe (6a) von dem Produktbehälter (6). Nach dem Entfernen der Kappe von der Injektionsvorrichtung verbleibt die Nadelschutzkappe (6a) in der Kappe (1). Die Nadelschutzkappe (5a) wird in der Kappe (1), insbesondere durch das Eingriffselement (1a) gehalten, wie in der Figur 5 dargestellt ist. Ferner ist die Drehhülse (2) zumindest teilweise von der entfernten Kappe aufgenommen. Danach kann der Benutzer mit der Injektionsvorrichtung wie in der Figur 6 dargestellt ist, wobei die Kappe von der Injektionsvorrichtung entfernt ist, eine Injektion tätigen.

### Bezugszeichen:

- 1: Haltehülse
- 1a: zweites Element
- 2: Drehhülse
- 2a: erstes Gegenelement
- 2b: zweites Gegenelement
- 3: Eingriffselement
- 4: Nadelschutzhülse
- 5: Gehäuse
- 5a: erstes Element
- 6: Produktbehälter
- 6a: Nadelschutzkappe
- 6b: Injektionsnadel
- 6c: Produktbehälterhalter
- L: Längsachse

## Patentansprüche

1. Injektionsvorrichtung mit einer Längsachse (L) mit:
- einem Gehäuse (5) zur Aufnahme eines Produktbehälters (6), wobei der Produktbehälter (6) eine fest verbundene Injektionsnadel (6b) aufweist, wobei an dem Produktbehälter (6) eine Nadelschutzkappe (6a) lösbar angeordnet ist, welche die Injektionsnadel (6b) umschliesst und gegenüber der Umgebung steril abdichtet,
- einer Kappe, welche lösbar an einem distalen Ende des Gehäuses (5) vorgesehen ist,
- wobei die Kappe ein Eingriffselement (3), eine Haltehülse (1) zum Greifen der Kappe, wobei die Haltehülse (1) relativ zu dem Gehäuse (5) axial bewegbar und drehfest angeordnet ist, und eine Drehhülse (2), welche zumindest teilweise von der Haltehülse (1) aufgenommen ist, umfasst, wobei die Drehhülse (2) relativ zu dem Gehäuse (5) und relativ zu der Haltehülse (1) drehbar angeordnet ist,
- wobei ferner das Eingriffselement (3) axialfest, insbesondere axialfest und drehbar zu der Drehhülse (2) und axial bewegbar zu dem Gehäuse (5) angeordnet ist, wobei das Eingriffselement (3) derart ausgebildet ist, um mit der Nadelschutzkappe (6a) im Eingriff sein zu können und um beim Entfernen der Kappe von der Injektionsvorrichtung das Entfernen der Nadelschutzkappe (6a) von dem Produktbehälter (6) zu bewirken,
- ein erstes Element (5a), welches an dem Gehäuse (5) vorsehen ist und welches in einem ersten Führungseingriff mit einem an der Drehhülse (2) vorgesehenen ersten Gegenelement (2a) ist,
- ein zweites Element (1a), welches an der Haltehülse (1) vorgesehen ist und welches in einem zweiten Führungseingriff mit einem an der Drehhülse (2) vorgesehenen zweiten Gegenelement (2b) ist, wobei ferner der erste und der zweite Führungseingriff derart ausgebildet sind, dass beim Entfernen der Kappe von der Injektionsvorrichtung das Entfernen der Nadelschutzkappe (6a) von dem Produktbehälter (6) bewirkt wird, **dadurch gekennzeichnet,**
- **dass** der erste und der zweite Führungseingriff als zueinander gegenläufige Gewindeeingriffe mit unterschiedlichen Steigungen ausgebildet sind.

2. Injektionsvorrichtung nach Anspruch 1, **dadurch gekennzeichnet, dass** das erste (5a) und/oder das zweite Element (1a) und/oder das erste (2a) und/oder das zweite Gegenelement (2b) als Führungskulisse und/oder Führungsnocke ausgebildet sind.

3. Injektionsvorrichtung nach Anspruch 1 oder 2, **dadurch gekennzeichnet, dass** die Steigung des zweiten Gewindeeingriffs (1a; 2b) grösser als die Steigung des ersten Gewindeeingriffs (5a; 2a) ist.

4. Injektionsvorrichtung nach Anspruch 1 oder 2 oder 3, **dadurch gekennzeichnet, dass** die Gewindeeingriffe, insbesondere der zweite Gewindeeingriff (1a; 2b) als nicht-hemmende Gewinde ausgebildet sind.

5. Injektionsvorrichtung nach einem der vorhergehenden Ansprüche, **dadurch gekennzeichnet, dass** die Haltehülse (1) und die Drehhülse (2) über den ersten und/oder den zweiten Führungseingriff derart gekoppelt sind, dass beim Entfernen der Kappe von der Injektionsvorrichtung die Haltehülse (1) einen längeren axialen Weg als die Drehhülse (2) und das Eingriffselement (3) macht.

6. Injektionsvorrichtung nach einem der vorhergehenden Ansprüche, **dadurch gekennzeichnet, dass** das Eingriffselement (3) einen oder mehrere Haken aufweist oder hakenförmig ausgebildet ist.

7. Injektionsvorrichtung nach einem der vorhergehenden Ansprüche, **dadurch gekennzeichnet, dass** das Eingriffselement (3) elastisch oder plastisch verformbar ist.

## Claims

1. Injection device having a longitudinal axis (L), comprising:
- a housing (5) for receiving a product container (6), wherein the product container (6) has a fixedly connected injection needle (6b), wherein there is detachably arranged on the product container (6) a needle protection cap (6a) which encloses the injection needle (6b) and seals the injection needle from the surroundings in a sterile manner,
- a cap which is detachably provided at a distal end of the housing (5),
- wherein the cap comprises an engagement element (3), a holding sleeve (1) for gripping the cap, wherein the holding sleeve (1) is arranged so as to be axially movable and rotationally fixed relative to the housing (5), and a rotary sleeve (2) which is at least partially received by the holding sleeve (1), wherein the rotary sleeve (2) is arranged so as to be rotatable relative to the housing (5) and relative to the holding sleeve (1),
- wherein furthermore the engagement element (3) is arranged so as to be axially fixed, in particular axially fixed and rotatable with respect to the rotary sleeve (2) and axially movable with respect to the housing (5), wherein the engagement element (3) is designed in such a way as to be able to engage with the needle protection cap (6a) and when the cap is removed from the injection device to cause the needle protection cap (6a) to be removed from the product container (6),
- a first element (5a) which is provided on the housing (5) and which is in a first guiding engagement with a first mating element (2a) provided on the rotary sleeve (2),
- a second element (1a) which is provided on the holding sleeve (1) and which is in a second guiding engagement with a second mating element (2b) provided on the rotary sleeve (2), wherein furthermore the first and the second guiding engagement are designed in such a way that when the cap is removed from the injection device, the needle protection cap (6a) is caused to be removed from the product container (6), **characterized**
- **in that** the first and the second guiding engagements are designed as mutually opposing threaded engagements with different pitches.

2. Injection device according to claim 1, **characterized in that** the first (5a) and/or the second element (1a) and/or the first (2a) and/or the second mating element (2b) are designed as a guide slot and/or a guide cam.

3. Injection device according to claim 1 or 2, **characterized in that** the pitch of the second threaded engagement (1a; 2b) is greater than the pitch of the first threaded engagement (5a; 2a).

4. Injection device according to claim 1 or 2 or 3, **characterized in that** the threaded engagements, in particular the second threaded engagement (1a; 2b), are designed as non-locking threads.

5. Injection device according to any of the preceding claims, **characterized in that** the holding sleeve (1) and the rotary sleeve (2) are coupled via the first and/or the second guiding engagement in such a way that when the cap is removed from the injection device, the holding sleeve (1) travels a longer axial path than the rotary sleeve (2) and the engagement element (3).

6. Injection device according to any of the preceding claims, **characterized in that** the engagement element (3) has one or more hooks or is hook-shaped.

7. Injection device according to any of the preceding claims, **characterized in that** the engagement element (3) is elastically or plastically deformable.

## Revendications

1. Dispositif d'injection comportant un axe longitudinal (L) comportant :
- un boîtier (5) pour la réception d'un récipient à produit (6), dans lequel le récipient à produit (6) présente une aiguille d'injection (6b) reliée de manière fixe, dans lequel un capuchon de protection d'aiguille (6a) est disposé de manière à être amovible sur le récipient à produit (6), lequel capuchon de protection d'aiguille entoure l'aiguille d'injection (6b) et rend celle-ci étanche de manière stérile vis-à-vis de l'environnement,
- un capuchon, lequel est prévu de manière à être amovible au niveau d'une extrémité distale du boîtier (5),
- dans lequel le capuchon comprend un élément de mise en prise (3), une douille de maintien (1) pour la saisie du capuchon, dans lequel la douille de maintien (1) est disposée de manière solidaire en rotation et mobile axialement par rapport au boîtier (5), et une douille rotative (2) qui est au moins partiellement reçue par la douille de maintien (1), dans lequel la douille rotative (2) est disposée de manière à pouvoir tourner par rapport au boîtier (5) et par rapport à la douille de maintien (1),
- dans lequel, en outre, l'élément de mise en prise (3) est disposé de manière axialement fixe, en particulier de manière axialement fixe et rotative par rapport à la douille rotative (2) et de manière axialement mobile par rapport au boîtier (5), dans lequel l'élément de mise en prise (3) est réalisé de manière à pouvoir venir en prise avec le capuchon de protection d'aiguille (6a) et, lors du retrait du capuchon du dispositif d'injection, de manière à provoquer le retrait du capuchon de protection d'aiguille (6a) du récipient à produit (6),
- un premier élément (5a) qui est prévu sur le boîtier (5) et qui est dans une première prise de guidage avec un premier contre-élément (2a) prévu sur la douille rotative (2),
- un second élément (1a) qui est prévu sur la douille de maintien (1) et qui est dans une seconde prise de guidage avec un second contre-élément (2b) prévu sur la douille rotative (2), dans lequel la première et la seconde prise de guidage sont en outre réalisées de telle sorte que, lors du retrait capuchon du dispositif d'injection, le retrait du capuchon de protection d'aiguille (6a) du récipient à produit (6) est provoqué,
**caractérisé en ce que**
- la première et la seconde prise de guidage sont réalisées sous forme de prises filetées tournant en sens inverse l'une par rapport à l'autre et présentant des pas différents.

2. Dispositif d'injection selon la revendication 1, **caractérisé en ce que** le premier (5a) et/ou le second élément (1a) et/ou le premier (2a) et/ou le second contre-élément (2b) sont réalisés sous forme de coulisses de guidage et/ou de rainures de guidage.

3. Dispositif d'injection selon la revendication 1 ou 2,
**caractérisé en ce que** le pas de la seconde prise filetée (1a ; 2b) est supérieur au pas de la première prise filetée (5a ; 2a).

4. Dispositif d'injection selon la revendication 1 ou 2 ou 3, **caractérisé en ce que** les prises filetées, en particulier la seconde prise filetée (1a ; 2b), sont réalisées sous forme de filets non bloquants.

5. Dispositif d'injection selon l'une des revendications précédentes, **caractérisé en ce que** la douille de maintien (1) et la douille rotative (2) sont accouplées par l'intermédiaire de la première et/ou de la seconde prise de guidage de telle sorte que, lors du retrait du capuchon du dispositif d'injection, la douille de maintien (1) effectue un déplacement axial plus long que la douille rotative (2) et l'élément de mise en prise (3).

6. Dispositif d'injection selon l'une des revendications précédentes, **caractérisé en ce que** l'élément de mise en prise (3) présente un ou plusieurs crochets ou est réalisé sous forme de crochet.

7. Dispositif d'injection selon l'une des revendications précédentes, **caractérisé en ce que** l'élément de mise en prise (3) est déformable élastiquement ou plastiquement.
